# EUROPÄISCHE PATENTSCHRIFT

(11) **EP 0 528 899 B1**
(45) Veröffentlichungstag und Bekanntmachung des Hinweises auf die Patenterteilung: **07.02.1996**
(21) Anmeldenummer: 91909255.1
(22) Anmeldetag: 08.05.1991
(51) Int. Cl.: C07C 219/08, C11D 1/62, D06M 13/463

(54) **QUATERNIERTE ESTER VON ALKANOLAMINEN MIT FETTSÄUREN UND VERWENDUNG ALS AVIVAGEMITTEL**
QUATERNERISED ESTERS OBTAINED FROM ALKANOL AMINES WITH FATTY ACIDS AND THEIR USE AS REVIVING AGENT
ESTERS QUATERNES OBTENUS A PARTIR D'ALKAMINES ET D'ACIDES GRAS, ET UTILISATION DE CES ESTERS COMME AGENT D'AVIVAGE

(30) Priorität: 17.05.1990 DE 4015849
(43) Veröffentlichungstag der Anmeldung: 03.03.1993
(73) Patentinhaber: Henkel Kommanditgesellschaft auf Aktien, D-40191 Düsseldorf (DE)
(72) Erfinder: UPHUES, Günter, D-4019 Monheim (DE); PLOOG, Uwe, D-5657 Haan (DE); JESCHKE, Rainer, W-4000 Düsseldorf 13 (DE); WALTENBERGER, Peter, D-5461 Breitscheid-Hollig (DE)
(86) Internationale Anmeldenummer: EP9100863
(87) Internationale Veröffentlichungsnummer: WO9117974

(56) Entgegenhaltungen:
- EP-A- 0 239 910
- EP-A- 0 284 036
- DE-A- 2 430 140
- DE-A- 3 608 093
- US-A-39 158 867
- Fette, Seifen und Anstrichmittel, Band 88, Oktober 1986, M. MÜNZING et al.: "Kinetik der Fetthärtung und Vergleich verschiedener Katalysatoren", Seiten 387-391, siehe den ganzen Artikel (in der Anmeldung erwähnt)

## Beschreibung

Gegenstand der Erfindung sind quaternierte Ester, erhältlich durch Umsetzung von ungesättigten Fettsäuren, die einen Gehalt von mindestens 40 Mol-% trans-konfigurierter Doppelbindungen aufweisen, oder deren Ester mit Alkanolaminen und anschließende Quaternierung der Reaktionsprodukte mit Alkylierungsmitteln sowie die Verwendung der Produkte als Wäscheweichspülmittel.

Quaternierte Ester aus gesättigten Fettsäuren und Alkanolaminen finden in der Textilavivage als Weichmacher Verwendung **[DE-A-16 19 058, DE-A-17 94 068]**. Eine besondere Bedeutung kommt dabei Produkten zu, die durch Umsetzung von 2 Mol gesättigter Fettsäure mit 1 Mol Triethanolamin und nachfolgende Quaternierung mit Dimethylsulfat oder Methylchlorid erhältlich sind. Textilien, die mit quaternierten Estern dieser Art behandelt wurden, verfügen zwar über einen guten Weichgriff, sie weisen jedoch häufig eine unerwünschte Hydrophopbie auf, die sich in einer schlechten Benetzbarkeit oder Wasseraufnahme der behandelten Gewebe äußert.

In der Vergangenheit hat es nicht an Versuchen gemangelt, dieses Problem zu lösen.

Aus der amerikanischen Patentschrift **US 39 15 867** ist z. B. bekannt, daß quaternierte Ester von Mischungen gesättigter und ungesättigter Fettsäuren, wie sie z. B. auf einfachem Wege durch Spaltung natürlicher Fette und Öle zugänglich sind, mit Triethanolamin ein gutes Avivagevermögen besitzen. In der Patentschrift wird ferner offenbart, daß quaternierte Ester auf Basis von ungesättigten Fettsäuren und Alkanolaminen über keinerlei textilweichmachende Eigenschaften verfügen. Gemische quaternierter Ester auf Basis von gesättigten und ungesättigten Fettsäuren weisen demnach schlechtere anwendungstechnische Eigenschaften als quaternierte Ester auf, zu deren Herstellung ausschließlich gesättigte Fettsäuren verwendet werden.

Quaternierte Triethanolamin-triester auf Basis gesättigter oder einfach ungesättigter Fettsäuren mit 12 bis 22 Kohlenstoffatomen sind aus der europäischen Patentanmeldung **EP-A-0 239 910** bekannt. Produkte dieser Art, die über drei lange Fettsäurereste und eine kurzkettige Alkylgruppe verfügen, zeigen jedoch unbefriedigende avivierende Eigenschaften.

Gemäß der Lehre der europäischen Patentanmeldung **EP-A-0 284 036** lassen sich schließlich technische Gemische von Estern gesättigter und ungesättigter Fettsäuren mit Glycerin, wie sie z. B. auf Basis von natürlichen Fetten und Ölen zugänglich sind, mit Alkanolaminen umestern und anschließend quaternieren. Da quaternierte Ester dieser Art wiederum ungesättigte Anteile enthalten, erweisen sie sich gesättigten Produkten im Hinblick auf den Weichgriff unterlegen.

Aufgabe der Erfindung war es somit, quaternierte Ester von Fettsäuren mit Alkanolaminen zu entwicklen, die in der Lage sind, Textilien sowohl einen guten Weichgriff, als auch eine hohe Hydrophilie zu vermitteln.

Gegenstand der Erfindung sind quarternierte Ester, erhältlich durch
A) Umsetzung von einfach ungesättigten Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen
   mit Alkanolaminen der Formel (I), 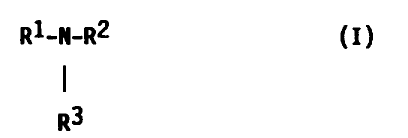 in der R¹ und R unabhängig voneinander Hydroxyalkylreste mit 2 bis 4 Kohlenstoffatomen darstellen und R³ für R¹ oder einen linearen oder verzweigten Alkylrest mit 1 bis 22 Kohlenstoffatomen steht und
B) anschließende Quaternierung der Reaktionsprodukte mit Alkylierungsmitteln.

Die Erfindung beruht auf der Erkenntnis, daß quaternierte Ester von Fettsäuren, die zu mindestens 40 Mol-% mit trans-konfigurierten Doppelbindungen vorliegen, mit Alkanolaminen Textilien bei der avivierenden Behandlung einen überraschend guten Weichgriff vermitteln und darüberhinaus weniger hydrophobierend wirken.

Als Fettsäuren für die Herstellung der erfindungsgemäßen quaternierten Ester kommen insbesondere die Elaidinsäure sowie elaidinsäurereiche technische Fettsäurefraktionen in Betracht, die einen Gehalt an Fettsäuren mit trans-konfigurierten Doppelbindungen von mindestens 40 Mol-% aufweisen.

Als Ausgangsmaterial für die Herstellung der quaternierten Ester kommen weiterhin Ester der vorgenannten Fettsäuren mit Alkoholen mit 1 bis 4 Kohlenstoffatomen in Betracht. Typische Beispiele sind Ethyl-, n-Propyl-, i-Propyl-, n-Butyl- oder insbesondere Methylester. In einer besonderen Ausführungsform der Erfindung können die Fettsäuren ferner als Voll- oder Partialester des Glycerins vorliegen.

Zur Herstellung der ungesättigten Fettsäuren mit einem Gehalt an trans-konfigurierten Doppelbindungen von mindestens 40 Mol-% oder deren Ester kann man von Linolsäure oder deren Estern ausgehen, die in Gegenwart von modifizierten Nickel-Katalysatoren selektiv zu Gemischen einfach ungesättigter Fettsäuren mit hohem Elaidinsäuregehalt ("trans-Gehalt") oder deren Ester hydriert werden. Eine andere Möglichkeit besteht darin, cis-Octadecensäure (Ölsäure) oder deren Ester in Gegenwart von Selen oder Salpetersäure zur trans-Octadecensäure (Elaidinsäure) oder deren Ester zu isomerisieren. Derartige Verfahren zur Herstellung von Elaidinsäure und Elaidinsäureester sind seit langem bekannt und z. B. in **Fette, Seif., Anstrichmitt., 88, 387 (1987)** oder **J.Am.Chem.Soc., 79, 4765 (1967)** beschrieben.

Wie in der Fettchemie üblich, können zur Herstellung der ungesättigten Fettsäuren mit einem Gehalt an trans-konfigurierten Doppelbindungen von mindestens 40 Mol-% oder deren Ester auch technische Gemische von Fettsäuren oder deren Ester eingesetzt werden, die auf Basis natürlicher Fette und Öle erhalten werden. In diesem Fall wird der trans-Gehalt von den in den technischen Gemischen enthaltenen gesättigten Anteilen begrenzt. Vorzugsweise geht man daher von Produkten aus, die einen hohen Gehalt an Linol- oder Ölsäure und einen geringen Anteil gesättigter Verbindungen aufweisen, wie z. B. Sonnenblumenöl oder Rüböl.

Quaternierte Ester mit besonders guten anwendungstechnischen Eigenschaften werden erhalten, wenn man technische Elaidinsäuregemische mit einem Gehalt von mindestens 40 Mol-%, vorzugweise mindestens 50 Mol-%, insbesondere mehr als 60 Mol-% Elaidinsäure, mit Alkanolaminen der Formel **(I)** umsetzt und die Reaktionsprodukte anschließend mit Alkylierungsmitteln quaterniert.

Als Alkanolamine kommen Di- und Trialkanolamine sowie deren Gemische in Betracht. Typische Beispiele sind Methyldiethanolamin, Ethyldiethanolamin, Kokosalkyldiethanolamin, Methyldiisopropanolamin oder Triethanolamin.

Quaternierte Ester mit besonders guten anwendungstechnischen Eigenschaften werden erhalten, wenn man die ungesättigten Fettsäuren mit Alkanolaminen der Formel **(I)**, in der R¹, R und R³ für einen Hydroxyethylrest stehen, umsetzt und die Reaktionsprodukte anschließend mit Alkylierungsmitteln quaterniert.

Gegenstand der Erfindung ist ferner ein Verfahren zur Herstellung von quaternierten Estern, bei dem man
A) einfach ungesättigten Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen mit Alkanolaminen der Formel (I), 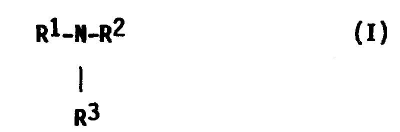 in der R¹ und R Hydroxyalkylreste mit 2 bis 4 Kohlenstoffatomen darstellen und R³ für R¹ oder einen linearen oder verzweigten Alkylrest mit 1 bis 22 Kohlenstoffatomen steht, umsetzt und
B) die Reaktionsprodukte anschließend mit Alkylierungsmitteln quaterniert.

Werden freie Fettsäuren in die Reaktion eingesetzt, findet mit den Alkanolaminen eine Veresterung statt. Die Reaktionspartner können dabei in einem molaren Verhältnis von Fettsäure zu Alkanolamin von 1,2 : 1 bis 2,5 : 1 eingesetzt werden. Quaternierte Ester mit besonders guten anwendungstechnischen Eigenschaften weisen im Durchschnitt zwei Estergruppen auf. Es ist daher von Vorteil, die Reaktion mit einem molaren Einsatzverhältnis von 1,5 bis 2,5, insbesondere 1,9 bis 2,2 durchzuführen.

Werden Ester der ungesättigten Fettsäuren eingesetzt, findet mit den Alkanolaminen eine Umesterung statt. Für die Einsatzmengen der Reaktionspartner gelten die oben genannten Bedingungen. Bei der Umesterung von Di- oder Triglyceriden mit Alkanolaminen beziehen sich die molaren Einsatzmengen auf die molare Menge der in den Fettsäureglycerinestern vorhandenen Fettsäureresten.

Die Veresterung der Fettsäuren mit den Alkanolaminen kann bei einer Temperatur von 150 bis 220°C durchgeführt werden. Eine optimale Reaktionsgeschwindigkeit wird erreicht, wenn die Reaktion im Bereich von 180 bis 200°C durchgeführt wird. Die Umsetzung wird dabei solange fortgesetzt, bis das Reaktionsprodukt eine Säurezahl kleiner 5 aufweist.

Die Umesterung der Fettsäureester mit den Alkanolaminen kann bei niedrigeren Temperaturen von 80 bis 220, vorzugsweise 80 bis 150°C, in Gegenwart von 0,1 bis 0,5 Gew.-% eines basischen Katalysators, z. B. Natriummethylat, durchgeführt werden. Der bei der Umsetzung gebildete Alkohol kann, wenn dies gewünscht wird, vor der Quaternierung der Ester durch Destillation entfernt werden.

Nach der Veresterung oder Umesterung wird das Rohprodukt einer Umsetzung mit Alkylierungsmitteln, insbesondere mit linearen oder verzweigten C₁₋C₃-Alkyl- oder C₇-C₁₀-Aralkylhalogeniden, -phosphaen oder -sulfaten wie z. B. Methylchlorid, Benzylchlorid, Trimehylphosphat oder vorzugsweise Dimethylsulfat unterworfen. Die Quaternierung kann in Substanz oder in Lösungsmitteln, wie z. B. Wasser oder niederen Alkoholen, bei Temperaturen von 60 bis 120, vorzugsweise 80 bis 100°C durchgeführt werden. Um sicherzustellen, daß das Quaternierungsprodukt frei von nichtabreagiertem Alkylieungsmittel ist, werden pro Mol Ester 0,7 bis 1,0 Mol Alkylierungsittel eingesetzt.

Besonders gute anwendungstechnische Eigenschaften weisen quaternierte Ester auf, in denen das quaternäre Stickstoffatom zwei langkettige und zwei kurzkettige Substituenten aufweist, beispielsweise Verbindungen, die durch Veresterung von Triethanolamin oder Methyldiethanolamin mit durchschnittlich zwei Mol ungesättigter Fettsäure und anschließende Quaternierung mit Dimethylsulfat erhalten werden.

Bei der avivierenden Behandlung von Textilien, wie beispielsweise Gewebe, Gewirke oder Garne, verleihen die quaternierten Ester den Stoffen einen guten Weichgriff und eine hohe Hydrophilie. Sie eignen sich daher als Avivagemittel für Textilien und als Wäscheweichspülmittel sowie zur Herstellung von Avivage- und Wäscheweichspülmitteln.

Die folgenden Beispiele sollen den Gegenstand der Erfindung näher erläutern.

### Beispiele

### I. Eingesetzte technische Fettsäuren

Fettsäure A wurde durch Selektivhydrierung von technischer Sonnenblumenfettsäure mit einem Gehalt von 62 Gew.-% Linolsäure in Gegenwart von modifizierten Nickelkatalysatoren gemäß dem in **Fette, Seif., Anstrichmitt., 88, 387 (1987)** beschriebenen Verfahren hergestellt. Fettsäure A wies nach der Selektivhydrierung einen trans-Gehalt von 62 Gew.-% auf. Zur Herstellung der Fettsäuren B - E mit trans-Gehalten von 50 bis 31 Gew.-%, wurde die Fettsäure A mit technischer Ölsäure oder Stearinsäure abgemischt. Die Kenndaten zu den eingesetzten Fettsäuren sind in Tab.1 zusammengefaßt.

**Tab.1:**

| Technische Fettsäuren | | | |
|---|---|---|---|
| Fettsäure | Säurezahl | Iodzahl | trans-Gehalt Gew.-% |
| A | 197,7 | 89,5 | 62 |
| B | 199,5 | 72,3 | 50 |
| C | 198,2 | 90,2 | 50 |
| | | | |
| D | 199,0 | 91,2 | 31 |
| E | 202,5 | 45,5 | 32 |
| | | | |

Die Produkte auf Basis technischer Fettsäuren mit einem trans-Gehalt von mindestens 40 Mol-% (A - C) sind erfindungsgemäß; Produkte auf Basis technischer Fettsäuren mit geringerem trans-Gehalt (D, E) dienen dem Vergleich.

### II. Herstellungsbeispiele

### Beispiel 1:

**Allgemeine Vorschrift zur Kondensation von Fettsäuren mit Triethanolamin**. In einem 1-1-Dreihalskolben mit Rührer, Innenthermometer und Rückflußkühler wurden 426 g (1,5 Mol) der Fettsäure A vorgelegt, auf 80°C erwärmt und mit 111,8 g (0,75 Mol) Triethanolamin versetzt. Die Reaktionsmischung wurde in 4 h auf 200°C erhitzt und 1 h bei gleicher Temperatur nachgerührt, wobei 27 g Reaktionswasser abgeschieden wurden. Nach Abkühlung auf 20°C wurden 509 g einer gelben Flüssigkeit erhalten, die eine Säurezahl von 4,7 und 2,0 Gew.-% titrierbarer Stickstoff aufwies.

250 g des Reaktionsproduktes wurden in einen 500 ml-Vierhalskolben mit Rührer, Innenthermometer, Tropftrichter und Rückflußkühler überführt und innerhalb von 1,5 h bei 80 bis 85°C mit 37,9 g Dimethylsulfat quaterniert. Die Reaktionsmischung wurde 1 h bei 85°C nachgerührt. Es wurden 387 g eines halbfesten Produktes erhalten, dessen Quaternierungsgrad - ermittelt durch Differenzbildung nach Zweiphasen-Titration im sauren und alkalischen Medium - 84 Gew.-% betrug. 80 g des quaternierten Produktes und 320 g Wasser wurden über 30 min bei 80°C zu einer feinteiligen Dispersion verrührt und anschließend auf 20°C abgekühlt (Produkt A*).

### Beispiele 2 und 3, Vergleichsbeispiele 1 und 2:

Beispiel 1 wurde unter Einsatz technischer Fettsäure mit hohem (Fettsäuren B und C, Beispiele 2 und 3) oder geringem trans-Gehalt (Fettsäuren D und E, Vergleichsbeispiele 1 und 2) wiederholt. Dabei wurden die Produkte B* bis E* erhalten.

### III. Anwendungstechnische Beispiele

Durch wiederholtes Waschen gehärtetes Baumwollgewebe (Molton) wurde unter Anwendung des Foulardverfahrens mit den Produkten der Beispiele 1 bis 3 sowie der Vergleichbeispiele 1 und 2 behandelt. Dabei galten folgende Vorgaben:
- Konzentration :: 30 g/l der 20 gew.-%igen Produkte
- Flottenaufnahme :: ca. 80 Gew.-% bezogen auf trockenes Gewebe
- Trocknung :: 3 min bei 180°C

Von den ausgerüsteten Prüfgeweben wurden die Wiederbenetzbarkeit und der Weichgriff beurteilt. Zur Prüfung der Wiederbenetzbarkeit diente der Steighöhentest nach **DIN 53 924**, wobei die Wassersteighöhe in mm nach 1 min bewertet wurde. Die Beurteilung des Weichgriffs erfolgte subjektiv durch 6 erfahrene Personen, die auf einer Skala von 0 = hart und rauh bis 6 = weich und volumniös Noten vergeben konnten. Die Testergebnisse sind in Tab.2 zusammengefaßt:

Unter den gleichen Bedingungen wurde Baumwoll-Frottiergewebe ausgerüstet. Die Beurteilung des Weichgriffs zeigte dem Moltongewebe vergleichbare Ergebnisse.

**Tab.2:**

| Wiederbenetzbarkeit und Weichgriff | | | |
|---|---|---|---|
| Bsp. | Fettsäure | Steighöhe mm | Griffnote |
| 1 | A* | 10 | 5,5 |
| 2 | B* | 5 | 5,5 |
| 3 | C* | 12 | 5,0 |
| | | | |
| V1 | D* | 0 | 5,5 |
| V2 | E* | 15 | 4,5 |
| | | | |

## Patentansprüche

1. Quarternierte Ester, erhältlich durch
A) Umsetzung von einfach ungesättigten Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen
mit Alkanolaminen der Formel (I), 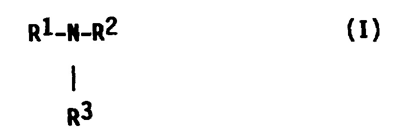 in der R¹ und R unabhängig voneinander Hydroxyalkylreste mit 2 bis 4 Kohlenstoffatomen darstellen und R³ für R¹ oder einen linearen oder verzweigten Alkylrest mit 1 bis 22 Kohlenstoffatomen steht und
B) anschließende Quaternierung der Reaktionsprodukte mit Alkylierungsmitteln.

2. Quaternierte Ester nach Anspruch 1, dadurch gekennzeichnet, daß technische Elaidinsäure oder deren Ester mit Alkanolaminen umgesetzt werden.

3. Quaternierte Ester nach einem der Ansprüche 1 und 2, dadurch gekennzeichnet, daß R¹, R und R³ in Formel (I) für einen Hydroxyethylrest stehen.

4. Verfahren zur Herstellung von quaternierten Estern, dadurch gekennzeichnet, daß
A) einfach ungesättigte Fettsäuren mit 16 bis 22 Kohlenstoffatomen und einem Gehalt von mindestens 40 Mol-% trans-konfigurierter Doppelbindungen oder deren Ester mit Glycerin oder aliphatischen Alkoholen mit 1 bis 4 Kohlenstoffatomen
mit Alkanolaminen der Formel **(I)**, 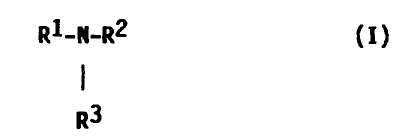 in der R¹ und R unabhängig voneinander Hydroxyalkylreste mit 2 bis 4 Kohlenstoffatomen darstellen und R³ für R¹ oder einen linearen oder verzweigten Alkylrest mit 1 bis 22 Kohlenstoffatomen steht, ungesetzt und
B) die Reaktionsprodukte anschließend mit Alkylierungsmitteln quaterniert werden.

5. Verfahren nach Anspruch 4, dadurch gekennzeichnet, daß technische Elaidinsäure oder deren Ester mit Alkanolaminen umgesetzt werden.

6. Verfahren nach einem der Ansprüche 4 und 5, dadurch gekennzeichnet, daß R¹, R und R³ in Formel **(I)** für einen Hydroxyethylrest stehen.

7. Verfahren nach mindestens einem der Ansprüche 4 bis 6, dadurch gekennzeichnet, daß die Umsetzung der ungesättigten Fettsäuren oder deren Ester mit den Alkanolaminen in einem molaren Verhältnis von 1,2 : 1 bis 2,5 : 1 durchgeführt wird.

8. Verfahren nach mindestens einem der Ansprüche 4 bis 7, dadurch gekennzeichnet, daß die Veresterung der ungesättigten Fettsäuren mit den Alkanolaminen bei einer Temperatur von 150 bis 220°C durchgeführt wird.

9. Verfahren nach mindestens einem der Ansprüche 4 bis 7, dadurch gekennzeichnet, daß die Umesterung der ungesättigten Fettsäureester mit den Alkanolaminen bei einer Temperatur von 80 bis 220°C durchgeführt wird.

10. Verfahren nach mindestens einem der Ansprüche 4 bis 9, dadurch gekennzeichnet, daß die Quaternierung mit Dimethylsulfat durchgeführt wird.

11. Verfahren nach mindestens einem der Ansprüche 4 bis 10, dadurch gekennzeichnet, daß die Quaternierung bei Temperaturen von 60 bis 120°C durchgeführt wird.

12. Verwendung der quaternierten Ester, erhältlich nach dem Verfahren nach mindestens einem der Ansprüche 4 bis 11 als Avivagemittel für Textilien oder Wäscheweichspülmittel.

13. Verwendung der quaternierten Ester, erhältlich nach dem Verfahren nach mindestens einem der Ansprüche 4 bis 11 zur Herstellung von Avivage- oder Wäscheweichspülmitteln.

## Claims

1. Quaternized esters obtainable by
A) reaction of unsaturated C₁₆₋₂₂ fatty acids containing at least 40 mol-% trans-configured double bonds or esters thereof with glycerol or aliphatic C₁₋₄ alcohols
with alkanolamines corresponding to formula **(I)** 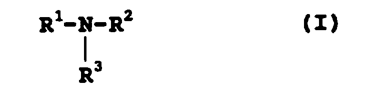 in which R¹ is a C₂₋₄ hydroxyalkyl group and R and R³ independently of one another have the same meaning as R¹ or represent a C₁₋₂₂ alkyl group, and
B) subsequent quaternization of the reaction products with alkylating agents.

2. Quaternized esters as claimed in claim 1, characterized in that technical elaidic acid or esters thereof are reacted with alkanolamines.

3. Quaternized esters as claimed in claim 1 or 2, characterized in that R¹, R and R³ in formula **(I)** represent a hydroxyethyl group.

4. A process for the production of quaternized esters, characterized in that
A) unsaturated C₁₆₋₂₂ fatty acids containing at least 40 mol-% trans-configured double bonds or esters thereof with glycerol or aliphatic C₁₋₄ alcohols
are reacted with alkanolamines corresponding to formula **(I)**, 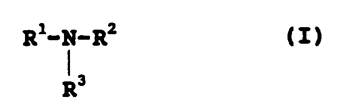 in which R¹ is a C₂₋₄ hydroxyalkyl group and R and R³ independently of one another have the same meaning as R¹ or represent a C₁₋₂₂ alkyl group, and
B) the reaction products are subsequently quaternization with alkylating agents.

5. A process as claimed in claim 4, characterized in that technical elaidic acid or esters thereof are reacted with alkanolamines.

6. A process as claimed in claim 4 or 5, characterized in that R¹, R and R³ in formula (I) represent a hydroxyethyl group.

7. A process as claimed in at least one of claims 4 to 6, characterized in that the reaction of the unsaturated fatty acids or esters thereof with the alkanolamines is carried out in a molar ratio of 1.2 : 1 to 2.5 : 1.

8. A process as claimed in at least one of claims 4 to 7, characterized in that the esterification of the unsaturated fatty acids with the alkanolamines is carried out at a temperature of 150 to 220°C.

9. A process as claimed in at least one of claims 4 to 7, characterized in that the transesterification of the unsaturated fatty acid esters with the alkanolamines is carried out at a temperature of 80 to 220°C.

10. A process as claimed in at least one of claims 4 to 9, characterized in that the quatnerization is carried out with dimethyl sulfate.

11. A process as claimed in at least one of claims 4 to 10, characterized in that the quaternization is carried out at temperatures of 60 to 120°C.

12. The use of the quaternized esters obtainable by the process claimed in at least one of claims 4 to 11 as fabric softeners.

13. The use of the quaternized esters obtainable by the process claimed in at least one of claims 4 to 11 for the production of fabric softeners.

## Revendications

1. Esters quaternisés accessibles par :
A) mise en réaction d'acides gras une fois non saturés ayant de 16 à 22 atomes de carbone et une teneur d'au moins 40 % molaire de doubles liaisons avec la configuration trans ou de leurs esters avec le glycérol ou des alcools aliphatiques ayant de 1 à 4 atomes de carbone, avec des alcanolamines de formule (I) 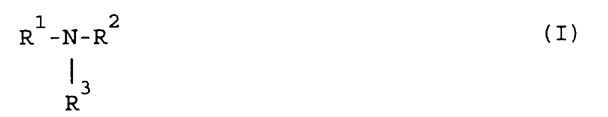 dans laquelle R¹ et R indépendamment l'un de l'autre, représentent des radicaux hydroxyalcoyle ayant de 2 à 4 atomes de carbone, et R³ représente R¹ ou un radical alcoyle linéaire ou ramifié ayant de 1 à 22 atomes de carbone et,
B) quaternisation consécutive des produits de réaction avec des agents d'alcoylation.

2. Esters quaternisés selon les revendications 1, caractérisés en ce que l'on fait réagir de l'acide élaïdinique industriel ou de ses esters, avec des alcanolamines.

3. Esters quaternisés selon la revendication 1 et 2, caractérisés en ce que R¹, R et R³ dans la formule (I) représentent un radical hydroxyéthyle.

4. Procédé d'obtention d'esters quaternisés, caractérisé en ce que :
A) on fait réagir des acides gras non saturés une fois, ayant de 16 à 22 atomes de carbone et ayant une teneur d'au moins 40 % molaire de doubles liaisons en configuration trans ou de leurs esters avec le glycérol ou avec des alcools aliphatiques ayant de 1 à 4 atomes de carbone,
avec des alcanolamines de formule (I) 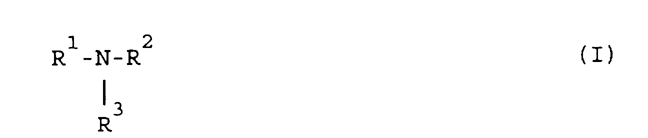 dans laquelle R¹ et R représentent des radicaux hydroxyalcoyle ayant de 2 à 4 atomes de carbone, et R³ représente R¹ ou un radical alcoyle linéaire ou ramifié avec de 1 à 22 atomes de carbone, et
B) on quaternise ensuite les produits de réaction avec des agents d'alcoylation.

5. Procédé selon la revendication 4, caractérisé en ce que de l'acide élaïdique industriel ou de ses esters, est mis à réagir avec des alcanolamines.

6. Procédé selon l'une des revendications 4 et 5, caractérisé en ce que R¹, R et R³ dans la formule (I) représentent un radical hydroxyalcoyle.

7. Procédé selon au moins une des revendications 4 à 6, caractérisé en ce que la réaction des acides gras non saturés ou de leurs esters, avec les alcanolamines est effectuée dans un rapport molaire de 1,2 : 1 à 2,5 : 1.

8. Procédé selon au moins une des revendications 4 à 7, caractérisé en ce que l'estérification des acides gras non saturés avec les alcanolamines est effectuée à une température allant de 150 à 220°C.

9. Procédé selon au moins une des revendications 4 à 7, caractérisé en ce que la transestérification des esters d'acide gras non saturés avec les alcanolamines est effectuée à une température allant de 80 à 220°C.

10. Procédé selon au moins une des revendications 4 à 9, caractérisé en ce que la quaternisation est effectuée avec du sulfate de diméthyle.

11. Procédé selon au moins une des revendications 4 à 10, caractérisé en ce que la quaternisation est effectuée à des températures allant de 60 à 120°C.

12. Utilisation des esters quaternisés accessibles selon le procédé selon au moins une des revendications 4 à 11, comme agent d'avivage pour les textiles ou les agents assouplissants pour le linge.

13. Utilisation des esters quaternisés accessibles selon le procédé, selon au moins une des revendications 4 à 11, pour l'obtention d'agents d'avivage ou d'agents assouplissants pour le linge.
